# EUROPEAN PATENT APPLICATION

(11) **EP 4 570 247 A1**
(43) Date of publication of application: **18.06.2025**
(21) Application number: 23216766.8
(22) Date of filing: 14.12.2023
(51) Int. Cl.: A61K 31/336, A61K 31/404, A61K 31/4725, A61K 31/635, A61K 45/06, A61P 35/00

(54) **COMBINED AGENTS WITH SYNERGISTIC EFFECT AGAINST GLIOMAS**

(71) Applicant: Eberhard Karls Universität Tübingen Medizinische Fakultät, 72074 Tübingen (DE)
(72) Inventor: TABATABEI, Ghazaleh, 72076 Tübingen (DE); HAEUSSER, Lara Annina, 72076 Tübingen (DE); KUHLBURGER, Laurence, 72070 Tübingen (DE); MERK, Daniel Josef Wolfgang, 71134 Aidlingen (DE)
(74) Representative: Witte, Weller & Partner Patentanwälte mbB

(57) **Abstract**

The present invention relates to a new pharmaceutical composition for the treatment and prophylaxis of a glioma comprising a first active agent combined with at least a second active agent, wherein the combination of said first and said second active agent results in a synergistic effect. The present invention also relates to a first active agent combined with at least a second active agent for use in the treatment and prophylaxis of a glioma wherein the combination of said first and said second active agent results in a synergistic effect. Finally, the invention relates to a method of treatment and prophylaxis of glioma in a subject in need, comprising the administration to the subject of said pharmaceutical composition or the combination of said first and said second active agent.

## Description

The present invention relates to a new pharmaceutical composition for the treatment and prophylaxis of a glioma comprising a first active agent combined with at least a second active agent, wherein the combination of said first and said second active agent results in a synergistic effect. The present invention also relates to a first active agent combined with at least a second active agent for use in the treatment and prophylaxis of a glioma wherein the combination of said first and said second active agent results in a synergistic effect. Finally, the invention relates to a method of treatment and prophylaxis of glioma in a subject in need, comprising the administration to the subject of said pharmaceutical composition or the combination of said first and said second active agent.

### FIELD OF THE INVENTION

The present invention relates to the field of drug development, more particular the field of active agents for the treatment and prophylaxis of a glioma.

### BACKGROUND OF THE INVENTION

Glioblastoma, previously known as glioblastoma multiforme (GBM), is the most aggressive and most common type of cancer that originates in the brain, and has very poor prognosis for survival. The cause of most cases of glioblastoma is not known. Uncommon risk factors include genetic disorders, such as neurofibromatosis and Li-Fraumeni syndrome, and previous radiation therapy. Glioblastomas represent 15% of all brain tumors. They are thought to arise from astrocytes.

Treatment usually involves surgery, after which chemotherapy and radiation therapy are used. The medication temozolomide is frequently used as part of chemotherapy. High-dose steroids may be used to help reduce swelling and decrease symptoms. Surgical removal (decompression) of the tumor is linked to increased survival, but only by some months.

VAL-083 is an alkylating agent that creates N7 methylation on DNA, with antitumor activity. Since a while VAL-083 is in discussion in respect of its potential to combat glioblastoma, however with but with ambiguous results. There is a tendency among experts to classify VAL-083 as having little or no effect in the treatment of glioblastoma; e.g., News release. Kintara Therapeutics. 31 October 2023; Guo et al. (2023), Phase 2 study of VAL-083 and radiotherapy in newly diagnosed MGMT-unmethylated GBM: case study reports. Presented at: 2023 European Association of Neuro-Oncology Annual Meeting; September 21-24, 2023; Rotterdam, Netherlands. Abstract P11.41.B; A trial to evaluate multiple regimens in newly diagnosed and recurrent glioblastoma (GBM AGILE). ClinicalTrials.gov. Updated 24 October 2023.

However, the compound VAL-083 cannot be denied an anti-glioblastoma potential and due to this potential and the high development costs it would be desirable to provide a new and improved use of VAL-083 that has the prospect of therapeutic success in the treatment and prevention of gliomas and in particular glioblastomas.

Against this background, it is the object of the present invention to find an improved application of VAL-083 in the treatment and prophylaxis of gliomas and glioblastomas.

### SUMMARY OF THE INVENTION

This object is met by the provision of a pharmaceutical composition for use in the treatment and/or prophylaxis of a glioma, said pharmaceutical composition comprising, in a therapeutically/prophylactically effective amounts, a first active agent combined with at least a second active agent, when administered to a subject, results in a synergistic effect, wherein said first active agent is VAL-083 (dianhydrodulcitol) and said second active agent is a Bcl-2 family inhibitor, in particular a Bcl-2 inhibitor and/or Bcl-xL inhibitor.

According to the invention "VAL-083" or dianhydrodulcitol or dianhydrogalactitol (CAS no. 23261-20-3), respectively, is an alkylating agent that creates N7 methylation on DNA, with antitumor activity; see Jiang et al. (2017), Dianhydrogalactitol, a potential multitarget agent, inhibits glioblastoma migration, invasion, and angiogenesis, Biomed. Pharmacother. 91:1065-1074.

The inventors used genome-wide CRISPR/Cas9 activation screens, thereby molecular targets were discovered which could potentiate VAL-083 effects. They validated selected druggable targets by functional assays *in vitro.* The validated targets can be addressed by inhibitors of B-cell lymphoma (Bcl), especially of Bcl-2 and Bcl-xL.

"Bcl-2 family" includes all members of the Bcl-2 family, especially 'Bcl-2' and 'Bcl-xL'. "Bcl-2" (B cell lymphoma 2) is the founding member of the Bcl-2 family of regulator proteins that regulate apoptosis, by either inhibiting (anti-apoptotic) or inducing (pro-apoptotic) apoptosis. Bcl-2 is localized to the outer membrane of mitochondria, where it plays an important role in promoting cellular survival and inhibiting the actions of pro-apoptotic proteins. The pro-apoptotic proteins in the Bcl-2 family, including Bax and Bak, normally act on the mitochondrial membrane to promote permeabilization and release of cytochrome c and ROS, that are important signals in the apoptosis cascade. These pro-apoptotic proteins are in turn activated by BH3-only proteins, and are inhibited by the function of Bcl-2 and its relative Bcl-xl. There are additional non-canonical roles of Bcl-2 that are being explored. "Bcl-xL" or'B-cell lymphoma-extra large' is a transmembrane molecule in the mitochondria. It is a member of the Bcl-2 family of proteins, and acts as an anti-apoptotic protein by preventing the release of mitochondrial contents such as cytochrome c, which leads to caspase activation and ultimately, programmed cell death. While the exact signaling pathway of Bcl-xL is still not known, it is believed that Bcl-xL differs highly from Bcl-2 in their mechanism of inducing apoptosis. Bcl-xL is about ten times more functional than Bcl-2 when induced by the chemotherapy drug, doxorubicin and can specifically bind to cytochrome C residues, preventing apoptosis. It can also prevent the formation of Apaf-1 and Caspase 9 complex by acting directly upon Apaf-1 rather than Caspase 9, as shown in nematode homologs.

"Bcl-2 family inhibitors" refer to a group of agents which can selectively and specifically target members of the Bcl-2 family and reduce or even block their enzymatic activities. They include, in particular, "Bcl-2 inhibitors" and "Bcl-xL inhibitors".

In conclusion, the inventors were able to demonstrate that the combination of the first and at least second active agents results in a synergistic effect with respect to the treatment or prophylaxis of a glioma. "Synergy" according to the invention means that the at least two active agents reinforce each other's effect such that the total effect of the active agents is stronger than a summation (potentiation or superadditive effect).

Methods to determine synergistic effects of at least two active agents are well known in the art, including the Bliss independence model and the Zero interaction potency (ZIP) model; see, e.g., Ma and Motsinger-Reif (2019), Current methods for quantifying drug synergism, Proteom Bioinform. 1(2): 43-48, and Yadav et al. (2015), Searching for drug synergy in complex dose-response landscapes using an interaction potency model, Comput. Struct. Biotechnol. J. 13: 504-513.

According to the invention, "prophylaxis" means any measure intended to prevent a disease or pathologic condition, such as a glioma, from developing or progressing to a pathogenic form.

According to the invention, "treatment" or therapy refers to all measures aimed at positively influencing or improving a disease or pathologic condition, such as a glioma.

According to the invention "therapeutically/prophylactically effective amount" or, synonymously, "effective dose", refer to the amount or dose of the first and/or at least second active agent, that is sufficient to achieve the desired clinical outcome or improvement.

A "subject" according to the invention refers to any living being, including a vertebrate, a mammal, and a human being.

According to the invention the first and/or second active agents also include the respective pharmaceutically acceptable salts thereof, the solvates thereof and the solvates of the salts thereof. Solvates for the purposes of the invention refer to those forms of the active agents, which in the solid or liquid state form a complex by coordination with solvent molecules. Hydrates are a specific form of solvates in which the coordination takes place with water.

In the pharmaceutical composition the first and second active agents can be, in an embodiment of the invention, the only active agents. "At least" a second active agent means that, in alternative embodiments, a third, fourth, fifth etc. active agent is comprised by the pharmaceutical composition according to the invention.

It goes without saying that the pharmaceutical compositing according to the invention may comprise a pharmaceutically acceptable excipient or carrier.

"Pharmaceutically acceptable excipients or carriers" are well known to the skilled person. They allow a proper formulation of the compound according to the invention and serve to improve the selectivity, effectiveness, and/or safety of drug administration. Pharmaceutically acceptable carriers include, without being limited thereto, solvents, fillers, binders, lubricants, stabilizers, surfactants, suspensions, thickeners, emulsifiers, preserving agents, liposomes, micelles, microspheres, nanoparticles, etc. suitable for the particular form of dosage. Except for cases, when the medium of conventional carriers is incompatible with the active ingredient, for example, upon occurrence of any undesirable biological effects or other adverse interactions with any other ingredient(s) of the pharmaceutical composition, the use of such compositions falls within the scope of this invention. Materials that can serve as pharmaceutically acceptable carriers include, but are not limited to, monosaccharides and oligosaccharides, as well as derivatives thereof; malt, gelatin; talc; excipients such as: cocoa butter and suppository waxes; oils, such as peanut oil, cotton-seed oil, safflower oil, sesame oil, olive oil, corn oil and soybean oil; glycols such as propylene glycol; esters, such as ethyl oleate and ethyl laurate; agar; buffering agents, such as magnesium hydroxide and aluminum hydroxide; alginic acid; pyrogen-free water; isotonic saline, Ringer's solution; ethyl alcohol and phosphate buffer solutions. In addition, the composition may contain other non-toxic compatible lubricants, for example sodium lauryl sulfate and magnesium stearate, as well as coloring agents, parting liquids, film formers, sweeteners, flavoring additives and flavorants, preserving agents and antioxidants.

The phrase "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

Suitable pharmaceutical acceptable carriers or excipients as well as pharmaceutical necessities for use in pharmaceutical formulations are described in Remington - The Science and Practice of Pharmacy, 23rd edition, 2020, a well-known reference text in this field, and in the USP/NF (United States Pharmacopeia and the National Formulary). Other sources are also available to one of ordinary skill in the art.

"Comprising" according to the invention generally means encompassing all the specifically mentioned features as well as optional, additional unspecified ones. However, according to embodiments of the invention, "comprising" also includes "consisting of" which means that only those features are included which are explicitly specified.

The problem underlying the invention is hereby completely solved.

According to an embodiment of the invention said Bcl-2 inhibitor is selected from the group consisting of: navitoclax (ABT-263), venetoclax (ABT-199), obatoclax (GX15-070), and ABT-737.

As the inventors were able to establish in their studies based on cell culture experiments, it is especially these specific Bcl-2 inhibitors in combination with VAL-083 that lead to a synergistic effect. For navitoclax this synergistic effect could even be demonstrated in additional experiment *in vitro.* These Bcl-2 inhibitors are therefore particularly well suited as second agents.

"Navitoclax" (previously ABT-263; CAS no.: 923564-51-6) is an anticancer drug which inhibits Bcl-2 along with Bcl-XL and Bcl-w proteins. In animal studies, navitoclax was found to be a senolytic agent, inducing apoptosis in senescent, but not non-senescent cells. Oral administration of ABT263 to either sublethally irradiated or normally aged mice reduced senescent cells, including senescent bone marrow hematopoietic stem cells and senescent muscle stem cells. This depletion mitigated total-body irradiation-induced premature aging of the hematopoietic system and rejuvenated the aged hematopoietic stem cells and muscle stem cells in normally aged mice. Navitoclax has been described in a combination treatment against solid tumors together with trametinib in a clinical trial. In this phase Ib/II study, patients with RAS-mutant tumors were enrolled to received trametinib plus navitoclax in dose-escalation part followed by multiple dose expansion cohorts.

"Venetoclax" (ABT-199; CAS no.: 1257044-40-8) is a medication used to treat adults with chronic lymphocytic leukemia (CLL), small lymphocytic lymphoma (SLL), or acute myeloid leukemia (AML). Venetoclax attaches to Bcl-2, thereby it blocks its actions and causes the death of cancer cells and slows down progression of the disease. Venetoclax is a BH3-mimetic. Venetoclax blocks the anti-apoptotic B-cell lymphoma-2 (Bcl-2) protein, leading to programmed cell death of CLL cells. Overexpression of Bcl-2 in some lymphoid malignancies has been linked to increased resistance to chemotherapy.

"Obatoclax" also known as obatoclax mesylate (GX15-070; CAS no.: 803712-67-6 or 803712-79-0 (mesylate)) is a drug for the treatment of various types of cancer. Obatoclax is an inhibitor of the Bcl-2 family of proteins. This inhibition induces apoptosis in cancer cells, preventing tumor growth. Several Phase II clinical trials were completed that investigated use of Obatoclax in the treatment of leukemia, lymphoma, myelofibrosis, and mastocytosis.

"ABT-737" (CAS no.: 852808-04-9) is a small molecule drug that inhibits Bcl-2 and Bcl-xL, two members of the Bcl-2 family of evolutionarily-conserved proteins that share Bcl-2 Homology (BH) domains. First developed as a potential cancer chemotherapy, it was subsequently identified as a senolytic (a drug that selectively induces cell death in senescent cells). ABT-737 was superior to previous BCL-2 inhibitors given its higher affinity for Bcl-2, Bcl-xL and Bcl-w. In vitro studies showed that primary cells from patients with B-cell malignancies are sensitive to ABT-737. In animal models, it improved survival, caused tumor regression, and cured a high percentage of mice. In preclinical studies utilizing patient xenografts, ABT-737 showed efficacy for treating lymphoma and other blood cancers.

The inventors have carried out most of their experiments - by way of example - with the Bcl-2 inhibitor navitoclax, but can conclude that other Bcl-2 inhibitors can also be used according to the invention in an advantageous manner, in particular those mentioned in the above embodiments.

In still another embodiment of the invention said Bcl-xL inhibitor is A-1331852 and/or A-1155463.

With this measure, specific Bcl-xL inhibitors are provided which, according to the inventors' findings, are particularly suitable as second active agents.

"A-1331852" (CAS no.: 1430844-80-6) is an orally available Bcl-xL selective inhibitor with a Ki of less than 10 pM.

"A-1155463" (Cas no.: 1235034-55-5) is a highly potent and selective Bcl-xL inhibitor, shows picomolar binding affinity to BCL-xL, and >1000-fold weaker binding to Bcl-2 and related proteins Bcl-W(Ki=19 nM) and Mcl-1(Ki>440 nM).

Each of the first and/or second active agent, in another embodiment, is present per dosage unit of the pharmaceutical composition at a concentration that, upon administration into a subject, results in a concentration in the glioma cells which is approx. ≤ 2000 µM, preferably about ≤ 1000 µM, further preferably ≤ 500 µM, further preferably ≤ 200 µM, further preferably ≤ 150 µM, further preferably ≤ 100 µM, further preferably ≤ 50 µM, further preferably ≤ 25 µM, further preferably ≤ 20 µM, further preferably ≤ 10 µM, further preferably ≤ 5 µM, further preferably ≤ 4 µM, further preferably ≤ 3 µM, further preferably ≤ 2.5 µM, further preferably ≤ 2 µM, and further preferably ≤ 1 µM.

This measure takes into account the findings of the inventors that setting the right therapeutic window by using the specified concentrations ensures on the one hand that the anti-glioma activity of the active agents is sufficient and on the other hand any cytotoxic effects are minimized.

In an embodiment of the invention the glioma addressed is a glioblastoma.

Glioblastoma, previously known as glioblastoma multiforme (GBM), is the most aggressive and most common type of cancer that originates in the brain, and has very poor prognosis for survival. There is no known method of preventing this cancer. Treatment usually involves surgery, after which chemotherapy and radiation therapy are used. However, the results achieved are often unsatisfactory and usually short-lived. Therefore, the invention provides a remedy here for the first time and provides an improved therapy and prophylaxis option.

In another embodiment of the invention the synergistic effect is determinable by the Bliss independence model and the Zero interaction potency (ZIP) model.

This measure has the advantage that the models are used which were also used by the inventors to find the combinations of active agents. They are therefore particularly suitable for determining the synergy. Both methods are well known to the skilled person; see, e.g., Ma and Motsinger-Reif *(loc. cit.)* and Yadav et al. (*loc. cit.*)*.*

Another subject-matter of the invention relates to a first active agent combined with at least a second active agent for use in the treatment and/or prophylaxis of a glioma, wherein the combination of said first and said second active agent, when administered to a subject, results in a synergistic effect, wherein said first active agent is VAL-083 (dianhydrodulcitol) and said second active agent is an Bcl-2 family inhibitor.

The embodiments, properties, advantages, and features of the pharmaceutical composition according to the invention apply in a corresponding way to the combination for use according to the invention.

A still further subject-matter of the invention relates to a method of treatment and/or prophylaxis of glioma in a subject in need, comprising the administration to said subject of the pharmaceutical composition according to the invention or the first active agent combined with at least a second active agent for use according to the invention.

The embodiments, properties, advantages, and features of the pharmaceutical composition according to the invention apply in a corresponding way to the method according to the invention.

In an embodiment of the method according to the invention the first and the second active agent are administered simultaneously. In another embodiment of the method according to the invention the first and the second active agent are administered separately.

Due to this method of administration, their respective benefits can be selectively exploited and external circumstances of treatment can be accommodated. Simultaneous administration facilitates administration and promotes patient compliance or adherence to therapy. Separate administration can induce a priming effect via the agent administered first.

It is to be understood that the before-mentioned features and those to be mentioned in the following cannot only be used in the combination indicated in the respective case, but also in other combinations or in an isolated manner without departing from the scope of the invention.

The invention is now further explained by means of embodiments and examples resulting in additional features, characteristics and advantages of the invention. The embodiments are of pure illustrative nature and do not limit the scope or range of the invention. The features mentioned in the specific embodiments are general features of the invention which are not only applicable in the specific embodiment but also in an isolated manner and in the context of any embodiment of the invention.

### BRIEF DESCRIPTION OF THE FIGURES

- Fig. 1:: CRISPR screen analyses using an activation library in GS-9, LN18, LN229, LNZ308 and T98G cells to identify resistance mechanisms of VAL-083. A: Scat-ter plot of MAGeCK MLE results from comparing Calabrese sgRNA distributions from DMSO or VAL-083-treated cells to the plasmid library pool merged for all cell lines. B: Rankview plot illustrating MAGeCK MLE results from comparing Calabrese sgRNA distributions from VAL-083-treated cells to the corresponding DMSO control merged for all cell lines.
- Fig. 2:: Acute cytotoxicity synergy assay results of combination treatment of VAL-083 with the Bcl2 family inhibitor navitoclax in LN229 (A), LN18 (B), and GS-9 (C).
- Fig. 3:: Acute cytotoxicity assay of a glioblastoma primary culture treated with VAL-083, navitoclax and a combination of both drugs for 72 h.

### EXAMPLES

### 1. Material and methods

### Acute cytotoxicity assay

The inventors seeded either 5,000 or 10,000 cells, treated them on the following day, and incubated them for 72 h. Following this incubation, the cell viability was assessed using CellTiterBlue reagent (Promega, Madison, Wl, US) in accordance with the manufacturer's instructions, utilizing a GloMax (Promega, Madison, Wl, US).

### Synergy assay

To assay drug combinations, the inventors treated the cells in 4x4 matrixes with VAL-083 and a second drug following the acute cytotoxicity assay protocol. The inventors analyzed the data for synergistic interactions with the R package synergyfinder to calculate zero interaction potential (ZIP) synergy scores for each tested combination in the 4x4 matrix and the average across the plate. Mean averages of two independent runs for each combination were then normalized over all combinations tested within a cell line. The highest average synergy value was set as 100% and a synergy value of 0 was set as 0%.

### Primary glioblastoma cultures

The inventors used fresh residual material obtained from primary tumor tissue after resection at the Department of Neurosurgery, University Hospital Tübingen, to obtain primary glioblastoma cultures (approved by the ethics committee of the University Hospital Tübingen, vote number 225/2023 BO2). The inventors cut the tissue into small pieces, washed with Hanks Balanced Salt Solution (HBSS) (Gibco, Carlsbad, CA, US), and then digested using collagenase and dispase (Roche, Basel, CH). Red blood cell lysis buffer (Sigma Aldrich, St. Louis, MO, US) was used to remove the remaining erythrocytes. For treatment, they followed the acute cytotoxicity assay protocol. The inventors treated the cells with VAL-083 and a second drug in monotherapy and combination. They then used the Bliss Independence Criterion to evaluate synergism potential. The inventors calculated a predicted value for additivity, the product of the two monotherapies, and compared it with the observed measurement of the combination treatment. Lower observed values compared to the prediction indicate towards synergy, higher towards antagonism.

### 2. Results

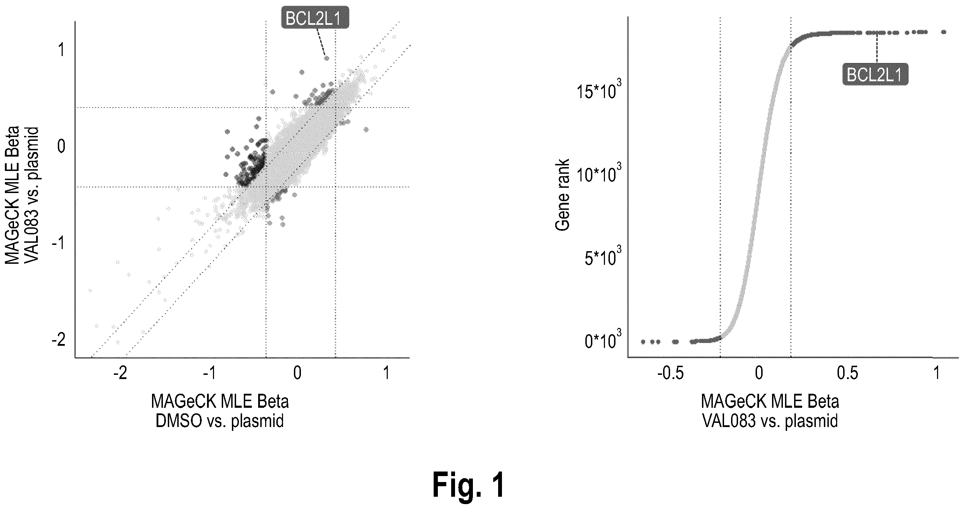

To identify mechanisms involved in VAL-083 treatment in glioblastoma, the inventors performed genome-wide CRISPR/Cas9 activation screens under treatment with VAL-083 or DMSO control. Activation screens (Figure 1) were performed in five glioma cell lines GS-9, LN229, LN18, LNZ308, and T98G using the Calabrese sgRNA library. Sequencing data was analyzed using MAGeCK MLE. Genes enriched in the VAL-083 treated cells but not in the DMSO treated cells confer resistance against treatment. These genes include the Bcl2 family member BCL2 like-1 (BCL2L1).

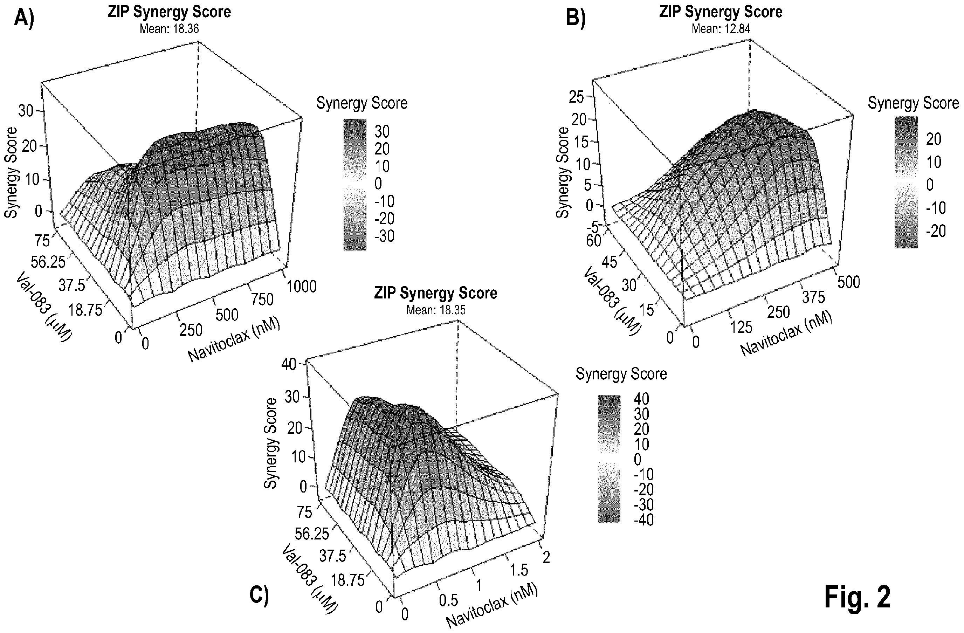

Therefore, the inventors combined VAL-083 treatment with drugs targeting Bcl2 family. The combination of VAL-083 with navitoclax was tested in a 72-hour acute cytotoxicity synergy assay in six glioma cell lines in two independent runs (Figure 2). The calculated ZIP synergy score for the combination treatments indicates a synergistic effect. The combination of VAL-083 with navitoclax was further validated in clonogenic survival assays with a synergistic effect of the combination according to the Bliss Independence Criterion.

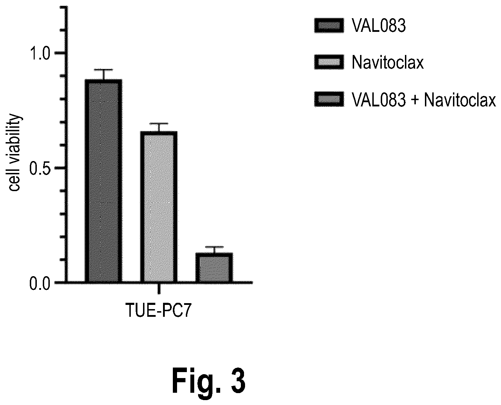

To further analyze the combination effect on cell viability of VAL-083 and navitoclax, the inventors treated a glioblastoma primary culture with both mono therapies and the combination. The observed effect of the combination treatment was synergistic compared to the predicted effect calculated from both monotherapies (Figure 3).

### 3. Conclusion

With the present invention, the inventors provide for the first time a combination of active ingredients comprising VAL-083 and a Bcl family inhibitor which, due to the observed clear synergistic effects of both components in the treatment and prophylaxis of gliomas, can be expected to lead to a significant therapeutic improvement over current treatment concepts. The inventors were able to demonstrate these effects in established experimental systems using representative example combinations.

## Claims

1. A pharmaceutical composition for use in the treatment and/or prophylaxis of a glioma, said pharmaceutical composition comprising, in a therapeutically/prophylactically effective amounts, a first active agent combined with at least a second active agent, when administered to a subject, results in a synergistic effect, wherein said first active agent is VAL-083 (dianhydrodulcitol) and said second active agent is a Bcl-2 family inhibitor.

2. The pharmaceutical composition for use of claim 1, wherein said Bcl-2 family inhibitor is navitoclax (ABT-263).

3. The pharmaceutical composition for use of claim 1 or 2, wherein said Bcl-2 family inhibitor is venetoclax (ABT-199).

4. The pharmaceutical composition for use of any of the preceding claims, wherein said Bcl-2 family inhibitor is obatoclax (GX15-070) and/or ABT-737.

5. The pharmaceutical composition for use of any of the preceding claims, wherein said Bcl-2 family inhibitor is A-1331852 and/or A-1155463.

6. The pharmaceutical composition for use of any of the preceding claims, wherein the glioma is a glioblastoma.

7. The pharmaceutical composition for use of any of the preceding claims, wherein the synergistic effect is determinable by Bliss independence model and the Zero interaction potency (ZIP) model.

8. A first active agent combined with at least a second active agent for use in the treatment and/or prophylaxis of a glioma, wherein the combination of said first and said second active agent, when administered to a subject, results in a synergistic therapeutic and/or prophylactic effect, wherein said first active agent is VAL-083 (dianhydrodulcitol) and said second active agent is an Bcl-2 family inhibitor.

9. The first active agent combined with at least a second active agent for use of claim 8, wherein said Bcl-2 family inhibitor is navitoclax (ABT-263).

10. The first active agent combined with at least a second active agent for use of claim 8 or 9 wherein said Bcl-2 family inhibitor is venetoclax (ABT-199).

11. The first active agent combined with at least a second active agent for use of any of the preceding claims, wherein said Bcl-2 family inhibitor is obatoclax (GX15-070) and/or ABT-737.

12. The first active agent combined with at least a second active agent for use of any of the preceding claims, wherein said Bcl-2 family inhibitor is A-1331852 and/or A-1155463.

13. A method of method of treatment and/or prophylaxis of glioma in a subject in need, comprising the administration to said subject of the pharmaceutical composition of any of claims 1-7 or the first active agent combined with at least a second active agent for use of any of claims 8-12.

14. The method of claim 13, wherein the first and the second active agent are administered simultaneously.

15. The method of claim 13, wherein the first and the second active agent are administered separately.
